# EUROPEAN PATENT APPLICATION

(11) **EP 1 854 475 A1**
(43) Date of publication of application: **14.11.2007**
(21) Application number: 06725763.4
(22) Date of filing: 15.02.2006
(51) Int. Cl.: A61K 38/22, A61P 3/06, A23L 1/29, C07K 14/575

(54) **USE OF LEPTIN FOR THE PREVENTION OF EXCESS BODY WEIGHT AND COMPOSITION CONTAINING LEPTIN**

(30) Priority: 23.02.2005 ES 200500408
(71) Applicant: Universitat de les Illes Balears, 07071 Palma de Mallorca (ES)
(72) Inventor: PALOU OLIVER, Andreu, E-07193 Bunyola (ES); PICÓ POU, Catalina, E-07015 Palma de Mallorca (ES); OLIVER VARA, Paula, E-07005 Palma de Mallorca (ES); SÁNCHEZ ROIG, Joana, Mallorca (ES); MIRALLES BORRACHINA, Olga, E-07190 Esporles Mallorca (ES)
(74) Representative: Ungria Lopez, Javier
(86) International application number: PCT/ES2006/000064
(87) International publication number: WO 2006/089987

(57) **Abstract**

The invention relates to the use of leptin in the preparation of an ingestible composition for children from nursing infants to adolescents, comprising leptin at a concentration greater than 50 ng/kg for the prevention of excess weight and/or the complications associated therewith. The invention comprises the use of a fully or partially hydrolyzed recombinant leptin. Said ingestible composition is a supplement for developmental nutrition, contains between 0.1 and 30 micrograms/liter leptin, provides a daily dose of leptin of more than 25 ng and can take the form o a solid, i.e, a tablet, capsule or reconstitutable powder, or a liquid, i.e., a syrup or drink.

## Description

### TECHNICAL FIELD OF THE INVENTION

The present invention belongs to the field of controlling body weight by means of compounds that prevent excess weight.

### STATE OF THE ART PRIOR TO THE INVENTION

Excess body weight is a disorder that is increasingly frequent in our developed societies, and in fact, it has become an important problem, not only in the esthetic sense, but also in the medical sense because it is a health problem and a source of other social and economic problems. Excess body weight is associated to higher risk for developing complications such as: respiratory conditions, cardiac conditions, diabetes, hypertension, etc.

Leptin is one of the most important signals that intervene in maintaining body weight, a circulating protein coded by the ob gene, a gene that was identified and cloned in 1994 [Zhang, Y., Proenca, R., Maffei, M., Barone, M., Leopold, L. and Friedman, J.M. (1994) Positional cloning of the mouse obese gene and its human homologue. Nature 372: 425-423] and that is expressed mainly in the adipose tissue. At the outset leptin was considered as an anti-obesity hormone, since animals with mutant ob genes, that is, animals whose ob genes lack leptin, develop obesity and diabetes. This is the case of the *ob*/*ob* mice that lack leptin and have an obese and diabetic phenotype. When leptin is administered to these mice their food intake decreases and their energy consumption increases, which in turn causes a decrease in body fat as well as in the circulating glucose and insulin. Therefore, leptin in these mice and in similar models, would have the function of regulating the size of fat deposits in the body, in fact, the plasmatic levels of this protein are directly related to the adipose mass, both in mice and men, however, administering leptin to obese humans is an ineffective practice in most cases.

It is known that the effect of leptin released in the bloodstream in an amount proportional to the adipose mass takes place at the central system level, mainly by signaling to the hypothalamus what are the levels of the fat reserves. Leptin produces then, based on a combination of the effect it has on the central system and on the peripheral system, a regulatory effect that is appropriate for the intake and the energy expenditure rates, helping the body to maintain body fat within a certain range. However, most obese humans seem to be resistant to the direct action of leptin, which is produced mainly in the adipose tissue. Administering this hormone, although it has proved to be effective in reducing body weight and normalize metabolic disorders in the ob gene, is not directly effective in the majority of obesity cases.

Nowadays we know that although adipocytes were initially believed to be the main source of leptin, this hormone can also be synthesized in other non-adipose tissues such as the placenta, mammary epithelium, skeletal muscle and the stomach [Smith-Kirwin, S.M., O'Connor, D. M., De Johnston, J., Lancey, E.D., Hassink, S.G. and Funanage, V. L. (1998). Leptin expression in human mammary epithelial cells and breast milk. J. Clin Endocrinol metab 83:1810-1813; Wang, J., Liu, R., Hawkins, M., Barzilai, N. and Rossetti, L. (1998). A nutrient-sensing pathway regulates leptin gene expression in muscle and fat. Nature 393: 684-688.]. Today we know that, aside from the its effect on the processes that regulate body weight, leptin has multiple other functions: detection of the fasting periods and enabling the pertinent metabolic adaptations, regulating reproduction and the immune system and interacting with other endocrine and neural mechanisms.

A noteworthy fact in what regards non-adipocyte leptin is that it is produced in considerable quantities in the stomachs of adult rats and humans, as well as by the mammary epithelium, since leptin is present in mother's milk [Casabiell, X. et al (1997). Presence of leptin in colostrums and/or breast milk from lactating mothers: a potential role in the regulation of neonatal food intake. J Clin Endocrinol Metab 82: 4270 -4273]. Leptin is also present in the stomachs of rodents during the prenatal and neonatal period, although it has not been synthesize endogenously, but supplied from the mother and comes, respectively from the amniotic liquid cells and the mammary gland cells that have released it for it to be absorbed by the immature gastric mucosa. In fact, it is not until the second week of life when endogenous leptin production from protein predominates over that obtained from external sources.

This data suggests that leptin has an important role in the early stages of neonatal life as a component of mother's milk, but we can say that to date, and despite its involvement in maintaining adequate control of body weight, no preventive effects of body weight gain have been demonstrated in the medium and long term after administration of leptin.

### DESCRIPTION OF THE INVENTION

The present invention refers to the use of leptin in the preparation of a composition for nursing infants and for children up to adolescence characterized in that it contains leptin in a proportion exceeding 50 ng/kg to prevent excess body weight and/or complications derived from said condition in later years. Said leptin is preferably a recombinant leptin that may have been originated by different species, including the human species, and can be a whole recombinant leptin or a partially hydrolyzed leptin. The expression "partially hydrolyzed leptin" refers to any fragment of said molecule that retains the functional properties of leptin.

Also, the present invention refers to the use of leptin in the preparation of a composition, said composition being a baby food product.

In addition, the present invention refers to the use of said ingestible composition characterized in that said composition is powder milk for nursing infants or a maternized milk formula for nursing infants. In a preferred embodiment of the invention, said ingestible composition is a paste, or baby meal, or follow-on formula. In these cases the ingestible composition contains a leptin concentration between 0.1 and 30 µg/ per liter of milk. In a preferred embodiment said leptin concentration is of 2.5 µg/ per liter.

On the other hand, using the ingestible composition object of the present invention is characterized in that the ingestible composition is a baby food product, or an infant nutritional supplement. In addition, the present invention is characterized in that said ingestible composition in any of its forms contributes a daily leptin dose ranging from 25 to 60000 ng. In a preferred embodiment of the present invention said nutritional supplement contributes a 1650 ng daily dose during the first month of life, 2300 ng daily during the second month of life and 2650 ng daily during the third and fifth months of life.

Said nutritional supplement has been selected, preferably from a liquid ingestible composition (syrup or drink) and a solid ingestible composition (orally ingestible tablet, orally ingestible capsule, or reconstitutable powder).

On the other hand, the present invention refers to an ingestible composition for nursing infants and children up to adolescence that includes leptin in a proportion exceeding 50 ng/kg of composition or is designed to contribute a daily dose of leptin greater than 25 ng.

In a particular embodiment of the present invention said composition is a child food product or powder milk for nursing infants or a maternized liquid milk formula for nursing infants or a paste or baby food or a follow-on milk formula. In a preferred embodiment of the invention said ingestible composition contains a leptin concentration between 0.1 and 30 µg/liter of milk and preferably of 2.5 µg/liter.

In another particular embodiment of the invention said composition is an infant nutritional supplement that contributes a daily dose of leptin of 25 to 60000 ng. Preferably, said nutritional supplement contributes 1650 ng daily of leptin the first month of life, 23000 ng daily during the second month of life and 2650ng daily during the third and fifth months of life.

In a preferred embodiment of the invention said infant nutritional supplement is liquid ingestible composition selected from a syrup and a drink, or a solid ingestible composition selected from a: compressed tablet, orally administered tablets, orally administered capsules and reconstitutable powder.

Otherwise, the ingestible composition includes leptin that is preferably a recombinant leptin that comes from several species, which can be either a whole recombinant leptin or a partially hydrolyzed leptin.

### EMBODIMENT OF THE INVENTION

The results obtained from mice used in laboratory experiments can be generalized to humans and other species. These results show, for the first time, that when leptin is administered orally during the nursing stage it is capable of protecting against weight gain during the adult stage (both in the case of consumption of a hypercaloric diet or a standard diet).

The results of the main experiments are detailed below:

### Experiment 1: study of immature gastric epithelium of leptin absorption and possible functional effects

A preliminary experiment consisted in administering a sole dose of leptin to neonatal rats during their nursing stage to check whether the leptin was absorbed and whether the leptin caused any physiological effect to the nursing rats, which would demonstrate that it was being absorbed in a functional manner. Later a different experiment was carried out that consisted in administering a dose of leptin to neonatal rats during their entire lactation period (21 days). After this period the neonatal rats were killed to verify whether ingesting leptin in an additional to that found in mother's milk caused any physiological effect during this early developmental stage, preferably on intake or on body weight.

During the first study, and to verify absorption of leptin, a sole oral dose of leptin was administered corresponding to 5 times the daily intake of leptin contained in mother's milk, to 4 day old rats, followed by taking blood and stomach samples before and after administering said dose. The concentration of leptin in both serum and stomach samples was determined by an ELISA test. One hour after administering an oral dose of leptin to 4 day old rats the levels of leptin in blood and stomach samples had increased (see Table 1), which shows that leptin is absorbed by immature gastric epithelium. Also, this leptin did cause an effect, since intake was seen to diminish.

**Table 1: weight of gastric contents and the amount of leptin found in blood serum and in the stomachs of 4 day old control rats and in rats treated with one sole dose of oral leptin 1 hour after administration. We used rats from 5 different litters and the values obtained from each litter as expressed as a percentage of the value of each of their controls. The results express the averages ± SEM.**

| • Statistical significance versus the value of the control rats | | |
|---|---|---|
| | **Control Rats** | **Rats treated with leptin** |
| Gastric content (%) | 99.93 ± 9.8 | 66.11 ± 11.54 * |
| Leptin in blood serum (%) | 100.05 ± 9.98 | 200.24 ± 20.96 * |
| Leptin in stomach (%) | 100.04 ± 16.73 | 173.11 ± 17.71* |

The neonatal rats used for the experiments were administered, during their nursing stage (that lasts the first 21 days after their birth) one oral dose of leptin equivalent to 5 times the daily intake obtained from mother's milk. These animals were killed at the end of their nursing period. Leptin was administered orally from day 1 to day 20 of the nursing period and during the two first hours of the light cycle, by pipette. The control rats received the excipient (water). The leptin used in the experiment was murinid recombinant leptin. The quantity of leptin administered to the animals was increased by 1 ng on day 1, to 43.8 ng on day 20. This dosage was calculated as 5 times the amount of leptin ingested daily from their mother's milk, which was in turn calculated from the concentration of leptin in maternal milk (a group of 10 nursing mothers was previously quantified at three different stages of lactation on days 7, 14 and 21) and from the estimated total daily milk intake during the nursing period as described in the bibliography [Kojima, T., Nishimura, M., Yajima, T., Kuwata, T., Suzuki, Y., Goda, T., Takase, S. and Harada, E. (1998). Effect of intermittent feeding on the development of disaccharidase activities in artificially reared rat pups. Comp Biochem Physiol A Mol Integr Phsyiol 121: 289-297].

Serum samples and the stomachs of these animals were obtained. The contents of endogenously produced leptin found in the stomachs of rat pups treated orally with exogenous leptin during the lactation period were lower than the amounts found in the stomachs of the control animals. The treated pups also had lower gastric contents. In what pertains to the gastric content of leptin we observed a significant reduction of 23% going from 1.080 ± 0.073 grams to 0.831 ± 0.031 g in treated rats. Body weight was not affected by treatment.

Therefore, we can conclude that leptin administered exogenously is absorbed in the stomach during neonatal development and it is capable of having an effect on the endogenous production of leptin, while in addition decreasing food intake. Therefore, it is feasible that it may influence body weight in adults.

### Experiment 2: study of the effect of leptin administration during the lactating period in the body weight of 12 week old adults.

After verifying that leptin administered orally during the lactation stage was absorbed in functional form by the immature stomachs of neonatal rats we performed a new experiment to determine a possible influence of ingesting an additional amount of leptin during lactation on the body weight evolution of adult animals.

Neonatal rats of the same litter were then adjusted to a total number of 10 that were randomly divided in two groups: a control group and a group treated with leptin. From day 1 to day 20 of the lactation period they were given 20 µl orally of the excipient (water) or of a murinid recombinant leptin by means of a pipette. The amount of leptin that was given to the animals was increased from 1 ng on day 1 to 43.8 ng on day 20 (see experiment 1). This dosage was calculated as five times the amount of leptin ingested daily from breastfed mother's milk, that was in turn calculated from the concentration of leptin found in maternal milk (a group of 10 nursing mothers was previously quantified at three different stages of lactation on days 7, 14 and 21) and from the estimated total daily milk intake during the nursing period as described in the bibliography [Kojima, T., Nishimura, M., Yajima, T., Kuwata, T., Suzuki, Y., Goda, T., Takase, S. and Harada, E. (1998). Effect of intermittent feeding on the development of disaccharidase activities in artificially reared rat pups. Comp Biochem Physiol A Mol Integr Phsyiol 121: 289-297]. We used male rats.

On day 21, after weaning, the control rats and the rats treated with leptin were divided in two groups: a normolipidic group that was fed with a diet of standard dry feed (3.8 Kcal/g) in which 10% of the calories present were derived from fat, and a hyperlipidic group fed with a dry feed diet (4.7 kcal/g) in which 45% of the calories present were derived from fat.

The body weight and food intake were recorded three times a week from the time of weaning for a period of 12 weeks total. The guidelines of the Universitat de le Illes Balears for the use and care of laboratory animals were followed at all times.

When the rats were 12 weeks old no significant effect of the leptin treatment during lactation was observed on the weight of adult rats in either group: rats that had been fed a normolipidic diet or the group of rats that have been fed a hyperlipidic diet (see Table 2 showing individual examples), and therefore we decided to continue recording weights for a longer period of time. It should be noted, however, that in some litters a certain tendency to weigh less was observed in the rats that had been treated with leptin.

**Table 2: body weight at 12 weeks of age of male control rats and of rats treated with leptin during their lactation period. Two specific individual examples of siblings from the same litter are shown (1-2 animals per group). The average obtained from animals of all five litters is also shown. The results are expressed as averages ± SEM.**

| Diet | **Treatment** | **Weight of Litter 1 (g)** | **Weight of Litter 2 (g)** | **Average weight of all 5 litters (g)** |
|---|---|---|---|---|
| Normolipidic | Control | 392 | 359 | 375 ± 14 |
| | leptin | 392 | 352 | 364 ± 13 |
| Hyperlipidic | Control | 416 | 415 | 412 ± 16 |
| | leptin | 414 | 377 | 392 ± 6 |

### Experiment 3: Study of the effect of having administered leptin during the lactation period on the body weight of 25 week old adult rats

Following the same experimental protocol described for experiment 2, the time assigned to control body weight of adult rats was extended to 25 weeks. At this age we could observe how the rats that had been treated with an additional amount of leptin during the neonatal stage had lower body weights than the control rats, both for the group that had consumed a normolipidic diet as the group that had consumed a hyperlipidic diet, although the effect was more pronounced in the rats that were fed a hyperlipidic diet (see Table 3 in which individual examples are shown). Taking into account the global data from all the rat litter studies, the decrease in body weight seen in the rats treated with leptin is of approximately 7%, for those given a normolipidic diet and about 8% for those given a hyperlipidic diet. This is a significant decrease, since it is a known fact that a small reduction in body weight (particularly in obese individuals) is beneficial in terms of improving secondary complications associated to excess fat.

It should be noted that in some rat litters the effect was more pronounced than in others, probably because of the leptin concentration already present in the mother's milk they nursed, and this suggests that the dosage administered during the experiment can be adjusted within margins.

**Table 3: body weight at 25 weeks of age of male control rats and of rats treated with leptin during their lactation period. Two specific individual examples of siblings from the same litter are shown (1-2 animals per group). The average obtained from animals of five litters is also shown. The results are expressed as averages ± SEM.**

| * Statistical significance versus the value obtained from control rats fed the same diet at as the treated rats. | | | | |
|---|---|---|---|---|
| Diet | **Treatment** | **Weight of Litter 1 (g)** | **Weight of Litter 2 (g)** | **Average weight of the 5 litters (g)** |
| Normolipidic | Control | 540 | 481 | 528 ± 21 |
| | leptin | 519 | 479 | 492 ± 17 |
| Hyperlipidic | Control | 595 | 564 | 572 ± 17 |
| | leptin | 525 | 506 | 526 ± 10 |

In short, and as shown by the results of the present invention, recombinant leptin ingested orally causes effects, having a possible role in the prevention of weight gain during the adult stage. In addition, we deduced that products derived from the partial digestion (hydrolysis) of leptin, since leptin enters the digestive cavity where it is partially digested.
It is postulated, therefore, the use of food preparations containing mammal recombinant leptins specific for the various species, both whole and partially hydrolyzed. It is also advisable to use leptin from the same species as the species that are going to consume the food preparations, although this recommendation may not be considered as a limitation to the scope of the invention, since it is possible to use leptin from a species in a food product intended for a different species.

Based on this assumption the following example serves to illustrate the present invention, although said example is in not in any way intended to restrict the scope of the invention.

### Example of milk supplemented by leptin:

The composition of "infant milk" (*in English in the original*) formulas is intended to satisfy the nutritional requirements of children during the first four or six months of their life. These infant milks have a total energetic content that ranges between 60 and 75 kcal/100 ml their constituents are: proteins, lipids, carbohydrates, mineral substances and vitamins as listed in the next table (see Official Journal of the European Communities N° L 175, 4. 7. 1991, p. 40-42 for the detailed composition).

| **Component** | **Energy - (minimum-maximum values)** | **Type** |
|---|---|---|
| Proteins | 2.25 - 3 g/100 Kcal | Cow's milk proteins |
| | | Soy protein |
| Lipids | 3.3 - 6.5 g/100 Kcal | Lactose |
| | | Maltose |
| | | Saccharose |
| | | Maltodextrins |
| | | Glucose syrup |
| | | starch |
| Mineral substances | 20 - 60 mg/100 Kcal | Sodium |
| | 60 - 145 mg/100 Kcal | Potassium |
| | 50 - 125 mg/100 Kcal | Chlorine |
| | 50 - -----mg/100 Kcal | Calcium |
| | 25 -90 mg/100 Kcal | Phosphorous |
| | 5 - 15 mg/100 Kcal | Magnesium |
| | 0.5 - 1 mg/100 Kcal | Iron |
| | 0.5 - 1 mg/100 Kcal | Zinc |
| | 20 - 80 µg/100 Kcal | Copper |
| | 5 µg/100 Kcal | iodine |
| Vitamins | 60-180 µg/100 Kcal | Vitamin A |
| | 1 -2.5 µg/100 Kcal | Vitamin D |
| | 40 - - µg/100 Kcal | Thiamine |
| | 60 - - µg/100 Kcal | Riboflavin |
| | 250 - - µg/100 Kcal | Nicotinamide |
| | 300 - - µg/100 Kcal | Pantothenic acid |
| | 35 - - µg/100 Kcal | Vitamin B6 |
| | 1.5 - - µg/100 Kcal | Biotin |
| | 4 - - µg/100 Kcal | Folic acid |
| | 0.1 - - µg/100 Kcal | Vitamin B12 |
| | 8 - - µg/100 Kcal | Vitamin C |
| | 4 - - µg/100 Kcal | Vitamin K |
| | 0.5 mg/g of linoleic acid | Vitamin E |

According to the present invention, leptin is added as a supplement to this milk formula during its formulation in 2500 ng/l concentration, that is, 2.5 µg/liter.

The same procedure is feasible for follow-on milk (*n English in the origina*) (see Official Journal of the European Communities N° L 175, 4. 7.1991, p. 4-44 for the detailed composition).

## Claims

1. Use of leptin in the preparation of an ingestible composition for nursing infants and children until adolescence, **characterized in that** is comprises leptin in a proportion above 50 ng/kg used to prevent excess body weight and/or its complications at any other age after lactation.

2. Use of leptin according to claim 1, **characterized in that** the leptin used is a recombinant leptin.

3. Use of leptin according to claim 2, **characterized in that** the leptin used is obtained from different species, amongst which is the human species.

4. Use of leptin according to claims 2 or 3, **characterized in that** the leptin used is a whole recombinant leptin.

5. Use of leptin according to claims 2 or 3, **characterized in that** the leptin used is a partially hydrolyzed recombinant leptin.

6. Use according to any of the claims 1 to 5, **characterized in that** said ingestible composition is an infant food product.

7. Use according to any of the claims 1 to 6, **characterized in that** said ingestible composition is a powder milk formula for nursing babies.

8. Use according to any of the claims 1 to 6, **characterized in that** said ingestible composition is liquid maternized milk for nursing infants.

9. Use according to any of the claims 1 to 6, **characterized in that** said ingestible composition is a paste or solid baby food or a follow-on formula.

10. Use according to any of the claims from 6 to 9, **characterized in that** said ingestible composition contains between 0.1 and 30 µg/liter leptin concentration.

11. Use according to claim 10, **characterized in that** said leptin concentration is of 2.5µg/liter.

12. Use according to claim 1, **characterized in that** said ingestible composition is a nutritional supplement for infants.

13. Use according to claims 6 to 12, **characterized in that** said composition contributes a daily intake of leptin between 25 to 60000 ng.

14. Use according to claim 13, **characterized in that** said ingestible composition is a nutritional supplement that contributes a daily intake of leptin of 1650 ng during the first month of life.

15. Use according to claim 13, **characterized in that** said nutritional supplement contributes a daily intake of leptin of 2300 ng during the second month of life

16. Use according to claim 13, **characterized in that** said nutritional supplement contributes a daily intake of leptin of 2650 ng between the third and fifth month of life

17. Use according to any of the claims from 12 to 16, **characterized in that** said infant nutritional supplement is selected from: a liquid ingestible composition, and a solid ingestible composition.

18. Use according to claim 17, **characterized in that** said liquid ingestible composition is selected between a syrup and a drink preparation.

19. Use according to claim 17, **characterized in that** said solid ingestible composition is selected between an orally ingested tablet, an orally ingested capsule and reconstitutable powder.

20. An ingestible composition for nursing infants and children up to adolescence **characterized in that** it comprises leptin in a proportion above 50 ng/kg or is designed to contribute a daily intake of leptin above 25 ng.

21. An ingestible composition according to claim 20 **characterized in that** the ingestible composition is a baby food product or a powder milk for nursing infants or a liquid maternized formula for nursing infants or a paste or solid baby food or a follow on formula.

22. An ingestible composition according to either claim 20 or claim 21, **characterized in that** said ingestible composition contains a concentration of leptin between 0.1 and 30 µg/liter.

23. An ingestible composition according to either claim 22, **characterized**
**in that** said ingestible composition contains a concentration of leptin of 2.5 µg/liter.

24. An ingestible composition according to any of the claims from 20 to 23, **characterized in that** said composition is an infant nutritional supplement.

25. An ingestible composition according to claim 24, **characterized in that** said nutritional supplement contributes a daily leptin intake between 25 and 60000 ng.

26. An ingestible composition according to either claim 24 or 25, **characterized in that** it contributes a daily leptin intake of 1650 ng during the first month of life, 2300 ng during the second month of life and 2650 between the third and fifth months of life.

27. An ingestible composition according to any of the claims from 23 to 26, **characterized in that** it is a liquid ingestible composition selected from a syrup and a drink.

28. An ingestible composition according to any of the claims from 23 to 26, **characterized in that** it is a solid ingestible composition selected from: compressed tablets, orally administered tablets, orally administered capsules and reconstitutable powder.

29. An ingestible composition according to any of the claims from 20 to 28 **characterized in that** said leptin is a recombinant leptin obtained from different species.

30. An ingestible composition according to claim 29, **characterized in that** said leptin is a whole recombinant leptin.

31. An ingestible composition according to claims 29 or 30, **characterized in that** said leptin is a partially hydrolyzed recombinant leptin.
